# EUROPÄISCHE PATENTANMELDUNG

(11) **EP 1 923 470 A1**
(43) Veröffentlichungstag der Anmeldung: **21.05.2008**
(21) Anmeldenummer: 07119619.0
(22) Anmeldetag: 30.10.2007
(51) Int. Cl.: C12Q 1/68

(54) **GNA 11 Polymorphismen**

(30) Priorität: 10.11.2006 DE 102006053138
(71) Anmelder: Eurofins Medigenomix GmbH, 82152 Martinsried (DE)
(72) Erfinder: Riemann, Kathrin, Dr., 45145 Essen (DE); Siffert, Winfried, 45884 Gelsenkirchen (DE)
(74) Vertreter: Cohausz & Florack

(57) **Zusammenfassung**

In einem vitro-Verfahren zur Vorhersage von Krankheitsrisiken, Krankheitsverläufen, Arzneimittelrisiken und zum Auffinden von Drug-Targets sucht man nach einer oder mehreren Genveränderung/en in der Promotorregion des Gens GNA11 und/oder im Intron 1 des Gens GNA11 auf dem Humanchromosom 19p13.3.

## Beschreibung

### TECHNISCHES GEBIET DER ERFINDUNG

Die Erfindung betrifft ein in-vitro Verfahren, in dem eine Genveränderung zu diagnostischen Zwecken, insbesondere zur Vorhersage von Krankheitsrisiken, Krankheitsverläufen und als Mittel zum Auffinden von Drugtargets, verwendet wird.

### TECHNOLOGISCHER HINTERGRUND

Alle Zellen des menschlichen Körpers verfügen über Membranrezeptoren an ihrer Oberfläche, über die alle Zellfunktionen gesteuert werden. Zu solchen Rezeptoren gehören die so genannten heptahelikalen Rezeptoren für Hormone, Neurotransmitter und Chemokine. Daneben gibt es eine Vielzahl von Rezeptoren für Wachstumsfaktoren und Rezeptoren mit intrinsischer Tyrosinkinaseaktivität, beispielsweise Rezeptoren für Insulin, Insulin-like Growth Factor, Epidermal Growth Factor, Platelet-derived Growth Factor und viele mehr. Weiterhin existieren viele Rezeptoren, die für die Regulation der Blutbildung verantwortlich sind, wie der Rezeptor für Erythropoietin. Über solche Rezeptoren werden unter anderem Zellwachstum, Motilität, Genexpression, Apoptose und Chemotaxis gesteuert. Die genannten Rezeptoren übermitteln ihre Signale ins Zellinnere über die Aktivierung sogenannter heterotrimerer G-Proteine. Diese G-Proteine bestehen aus einer großen Familie unterschiedlicher Isoformen und sind jeweils aus unterschiedlichen α-, β- und γ-Untereinheiten zusammengesetzt. Derzeit sind 5 β-Untereinheiten, 13 γ-Untereinheiten und mehr als 20 α-Untereinheiten bekannt, die durch unterschiedliche Gene kodiert werden (Farfel Z et al.,The expanding spectrum of G protein diseases. N Engl J Med. 1999 Apr 1;340(13):1012-20). Durch die Kombination aus diesen verschiedenen α-, β- und γ-Untereinheiten entsteht eine Vielzahl unterschiedlicher heterotrimerer G-Proteine.

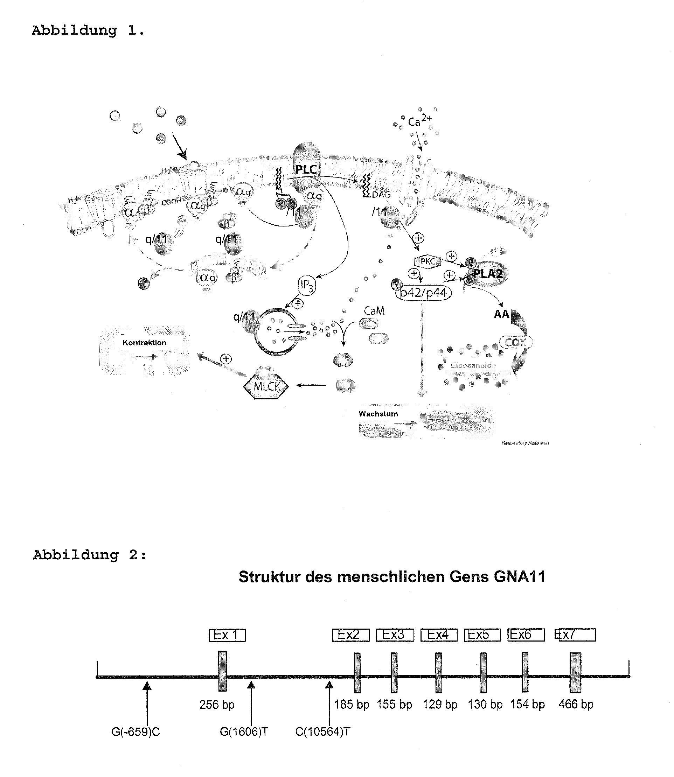

Dabei determiniert die Isoform-Kombination, welches Heterotrimer durch einen bestimmten Rezeptor aktiviert werden kann. Die βγ-Untereinheiten sind funktionell als Monomer zu betrachten. Im Ruhezustand hat die α-Untereinheit GDP gebunden (Abbildung 1). Nach Aktivierung eines koppelnden Rezeptors setzt die α-Untereinheit GDP im Austausch gegen GTP frei, und es kommt zur Dissoziation der βγ-Untereinheiten von den α-Untereinheiten. Sowohl die freien α- als auch die βγ-Untereinheiten können die Aktivität einer Vielzahl unterschiedlicher Effektoren steuern. Hierzu gehören beispielsweise Ionenkanäle, die Adenylylzyklase, die PI3-Kinase, unterschiedliche MAP-Kinasen. Die α-Untereinheiten verfügen über eine intrinsische GTPase-Aktivität die das nach Aktivierung gebundene GTP zu GDP hydrolysiert. Nachfolgend reassoziieren die freigesetzten βγ-Untereinheiten wieder mit der α-Untereinheit, wodurch der Aktivierungszyklus beendet wird. Damit steht das Heterotrimer für einen erneuten Aktivierungszyklus zur Verfügung (Bourne HR. How receptors talk to trimeric G proteins. Curr Opin Cell Biol. 1997;9(2):134-42).

Die Gα₁₁-Untereinheit wird in allen Körperzellen des Menschen exprimiert. Ihre Stimulation führt beispielsweise zur Aktivierung der Phospholipase C und damit zu einem Anstieg der intrazellulären Ca²⁺-Konzentration. Damit können beispielsweise Ca²⁺-abhängige Prozesse aktiviert werden. Ferner kann Gα₁₁ die Aktivität von Ionenkanälen, beispielsweise von Kalium- oder Calciumkanälen, regulieren. Nahezu alle bekannten Rezeptoren koppeln an Gα₁₁, so die Rezeptoren für Adenosin, Adrenalin, Angiotensin, Bradykinin, Calcitonin, Dopamin, Endothelin, Histamin, Noradrenalin, P2-purinerge Rezeptoren, Thrombin, Thromboxan, Vasopressin und viele mehr. Nach Stimulation Gα₁₁-gekoppelter Rezeptoren wird in vielen Zelltypen Apoptose induziert, so dass sich ein Zusammenhang zu Tumorerkrankungen und deren Verlauf und Therapieansprechen, aber auch ein Zusammenhang zu entzündlichen und immunologischen Erkrankungen und deren Verlauf und Therapieansprechen ergibt. Daneben werden vielfältige Stoffwechselwege durch Gα₁₁ reguliert.
Es ist von medizinisch großer Relevanz, Prognosefaktoren für den Verlauf von Krebserkrankungen zu definieren, die Auskunft über das Ansprechen auf bestimmte Therapieformen geben oder die prädiktiv für das Auftreten von Metastasen, die Tumorprogression und das Überleben sind. Bislang werden in der Medizin dem Fachmann allgemein bekannte Prognosefaktoren eingesetzt. Hierzu gehören beispielsweise die Größe des Tumors, seine Eindringtiefe in das umgebende Gewebe, organüberschreitendes Wachstum, das Eindringen in Blut- oder Lymphgefäße oder in Lymphknoten, sowie der Differenzierungsgrad der Tumorzellen. Daneben existieren einige relativ unspezifische serologische Marker. Das Verfahren zur Klassifikation der Tumore wird generell als "Staging" und "Grading" bezeichnet. Generell gilt, dass das Vorhandensein von Fernmetastasen und ein geringer Differenzierungsgrad prognostisch sehr ungünstige Parameter darstellen. Dennoch ist es die allgemeine Erfahrung in der Medizin, dass Patienten bei gleichem Tumorstadium drastisch unterschiedliche Krankheitsverläufe aufweisen. Während man bei manchen Patienten eine schnelle Progression der Erkrankung und das Auftreten von Metastasen und Rezidiven beobachtet, kommt die Erkrankung bei anderen Patienten aus unklaren Gründen zum Stillstand. Metastasen können hierbei lokal, regional und fern der Muttergeschwulst auftreten. Dazu ist es notwendig, dass eine hohe Zahl von Geschwulstzellen über den Lymph- oder Blutweg durch einfache Fortschwemmung in Flüssigkeit oder durch Abklatschen in benachbartes Gewebe gelangt. Unter Rezidivneigung versteht man das erneute Auftreten einer Geschwulst nach operativer unvollständiger oder Teilentfernung des Tumors. Hierbei handelt es sich nicht um eine erneute maligne Transformation, sondern um das Nachwachsen eines nicht vollständig entfernten Tumorgewebes. Eine Rezidivbildung ist auch durch Metastasen möglich, die viele Jahre latent bleiben können. Unter Progression versteht man das Wiederauftreten eines Tumors mit höherem Grading (mehr Entdifferenzierung) oder das Neuauftreten von Metastasen.

Ganz offensichtlich gibt es eine Vielzahl individueller, unerkannter biologischer variablen die den Verlauf einer Tumorerkrankung unabhängig von Staging und Grading in hohem Masse determinieren. Zu solchen Faktoren gehören genetische Wirtsfaktoren. Daneben ist es wünschenswert, genetische Marker zu entwickeln, die prädiktiv für das Auftreten von Tumoren sind. Solche Marker erfüllen die Funktion, die betroffenen Individuen frühzeitig weiteren Screening-Maßnahmen (Serologie, Röntgen, Ultraschall, NMR) zuzuführen. Damit können Krebserkrankungen im Frühstadium erkannt und therapeutisch angegangen werden, wobei die Heilungs- und Überlebenschancen bei Tumoren im Frühstadium wesentlich besser sind als bei fortgeschrittenen Tumoren.

### ZUSAMMENFASSUNG DER ERFINDUNG

Aufgabe der Erfindung ist es,
(a) funktionsverändernde genomische Polymorphismen und Haplotypen im Gen GNA11 bereitzustellen, die entweder zu einem Aminosäurenaustausch führen, oder
(b) die zur Änderung der Proteinexpression führen, oder
(c) die zum Auffinden und/oder Validieren weiterer Polymorphismen oder Haplotypen im Gen GNA11 geeignet sind.
(d) Nukleotidaustausche und Haplotypen bereitzustellen, die geeignet sind, generell Krankheitsrisiken- und Verläufe vorherzusagen,
(e) Nukleotidaustausche und Haplotypen bereitzustellen, die geeignet sind, generell Ansprechen auf Pharmaka und Nebenwirkungen vorherzusagen,
(f) Nukleotidaustausche und Haplotypen bereitzustellen, die geeignet sind, generell die Wirkung anderer Therapieformen vorherzusagen wie Bestrahlung, Wärme, Hitze, Kälte, Bewegung.

Gelöst werden diese Aufgaben durch ein *in vitro*-Verfahren, in dem man zur Vorhersage von Krankheitsrisiken, Krankheitsverläufen, Arzneimittelrisiken und zum Auffinden von Drug-Targets nach einer Genveränderung in der Promotorregion des Gens GNA11 und/oder in Intron 1 des Gens GNA11 des Humanchromosoms 19p13.3 sucht.

### ABBILDUNGEN

Abbildung 1 zeigt den Gα11 Signalweg. Das Diagramm zeigt wie der Gäll-Weg nach Rezeptorstimulation mit vielfältigen Signaltransduktionskomponenten verbunden ist, einschließlich Ionenkanäle, Transkriptionsfaktoren und Synthese von Eicosanodien. PLC, Phospholipase C; IP3, Inositoltrisphophat; PKC, Proteinkinase C; PLA2, Phospholipase A2; AA, Arachidonsäure; MLCK, Myosin Light Chain Kinase; CaM, Calmodulin; p42/p44-MAP-Kinase.

Abbildung 2 zeigt die Intron/Exon-Struktur des humanen GNA11 und Position des G(-659)C -Promotor-Polymorphismus sowie der Intron I Polymorphismen G(1606)T und C(10564)T (nicht maßstabsgetreu).

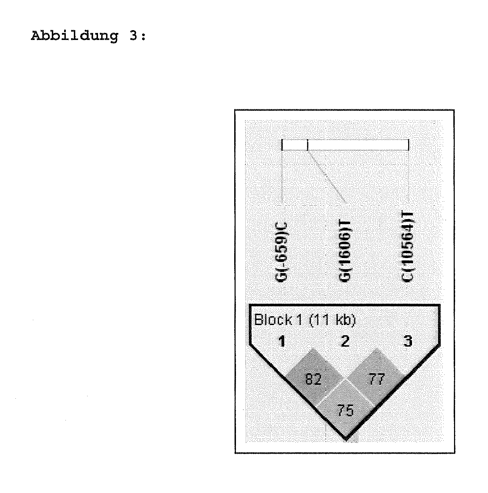

Abbildung 3 zeigt eine Haplotypenanalyse des Promotor-Polymorphismus G(-659)C (rs11084997)mit den IntronI-Polymorphismen G(1606)T und C(10564)T (rs3968546).

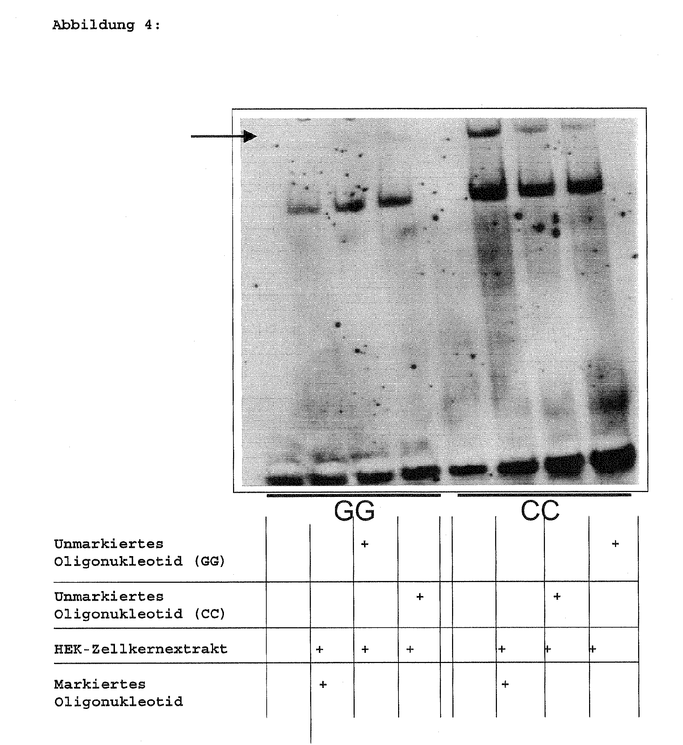

Abbildung 4 zeigt die Ergebnisse des Electrophoretic Mobility Shift Assays (EMSA) mit Konstrukten, die den (-659)GG oder den CC-Genotyp im Promoter von GNA11 enthalten. Nach Zugabe von Zellkernextrakt erkennt man eine vermehrte Bindung von Kernprotein and das "CC-Konstrukt", das eine weitere Bindungsstelle für den Transkriptionsfaktor AP2-alpha aufweist.

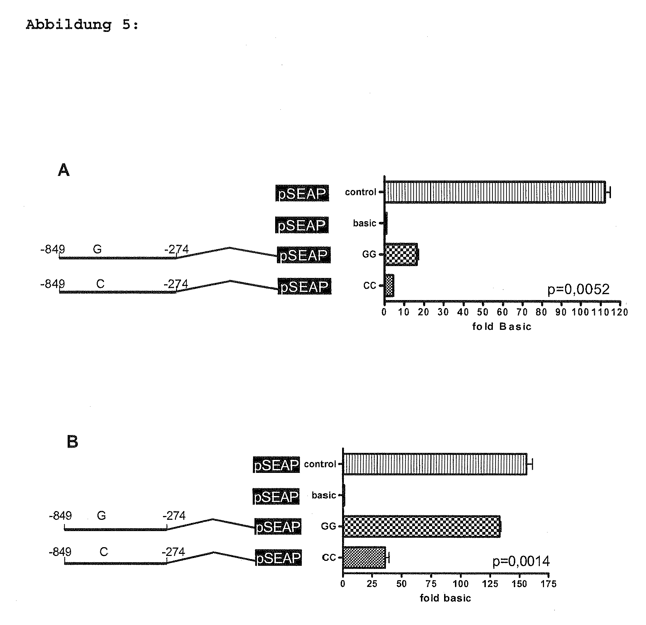

Abbildung 5 zeigt die genotypabhängige Aktivität des GNA11-Promotors. Das Promotorkonstrukt -849/-274 mit jeweils dem GG- oder dem CC-Genotyp wurde in HEK-und HELA-Zellen transfiziert. Die Sekretion von alkalischer Phosphatase wurde nach verschiedenen Zeiten gemessen. Man erkennt eine signifikant gesteigerte Aktivierung des Promotors mit dem GG-Genotyp sowohl bei HELA-Zellen (A: hier 12h nach Transfektion) als auch bei HEK-Zellen (B: hier 8h nach Transfektion).

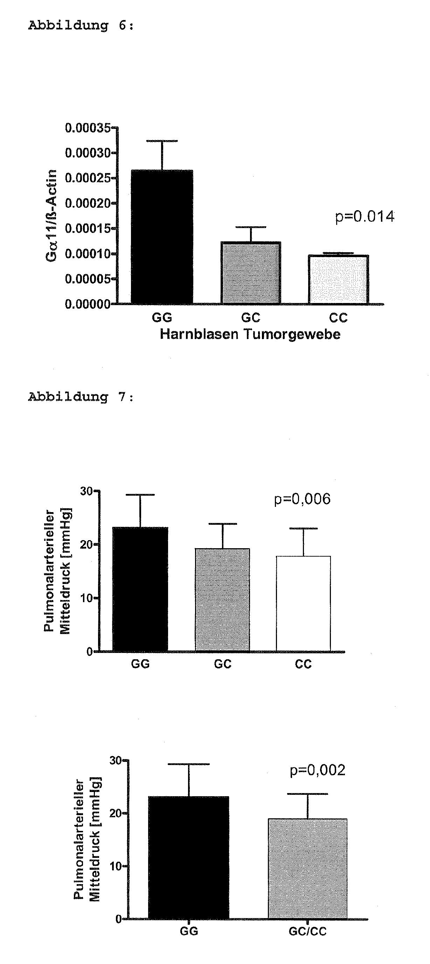

Abbildung 6 zeigt die Expression von Gα11 mRNA in Gewebe in Abhängigkeit vom G(-659)C-Polymorphismus. Dargestellt ist der Quotient Gα11/â-Actin mRNA.

Abbildung 7 zeigt den GNA11 G(-659)C- Polymorphismus und Kreislaufparameter bei Patienten. Dargestellt ist der pumonalarterielle Mitteldruck (oben) in Abhängigkeit vom Genotyp und die heterozygoten GC- mit den homozygot CC-Genotypen zusammengefaßt (unten).

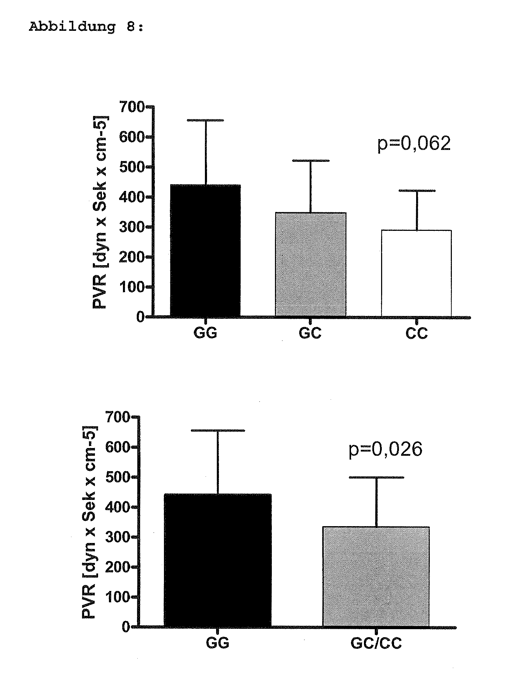

Abbildung 8 zeigt den GNA11 G(-659)C- Polymorphismus und Kreislaufparameter bei Patienten vor der Operation. Dargestellt ist der pumonalvaskuläre Widerstand (oben) in Abhängigkeit vom Genotyp und die heterozygoten GC- mit den homozygot CC-Genotypen zusammengefasst (unten).

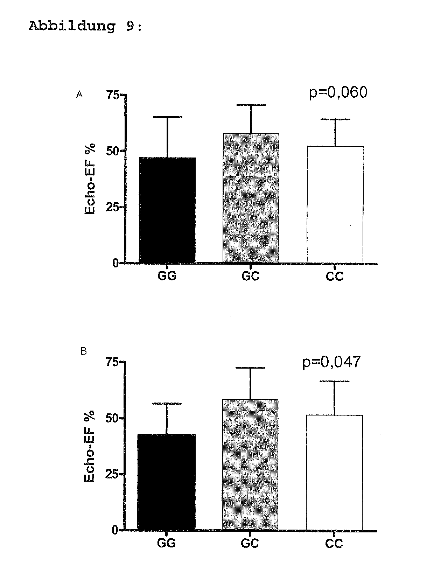

Abbildung 9 zeigt den GNA11 G(-659)C- Polymorphismus und die Echokardiographie bei Personen mit koronarer Herzerkrankung. Dargestellt ist die Auswurfsfraktion in % bei allen untersuchten Personen vor der Operation (A) und bei Personen mit Herzinfarkt (B) in Abhängigkeit vom Genotyp.

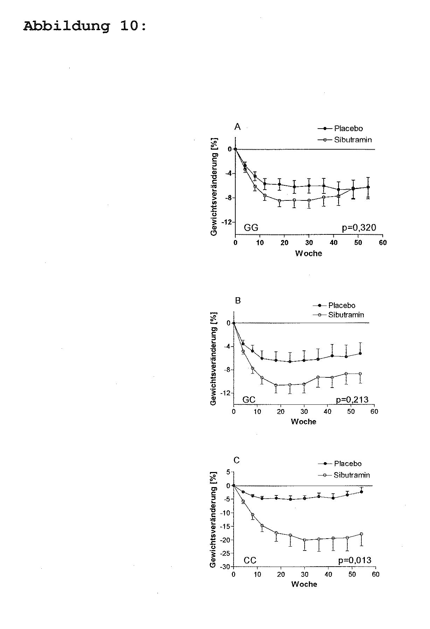

Abbildung 10 zeigt Zeitverläufe der Gewichtsveränderungen unter Placebo bzw. Sibutramin für den GNA11 (-659) GG Genotyp (A), den GC-Genotyp (B) und den CC-Genotyp (C). Man sieht, dass nur der CC-Genotyp von einer Therapie mit 15 mg Sibutramin täglich profitiert, während die GG-/GC-Genotypen ohne Medikament abnehmen können.

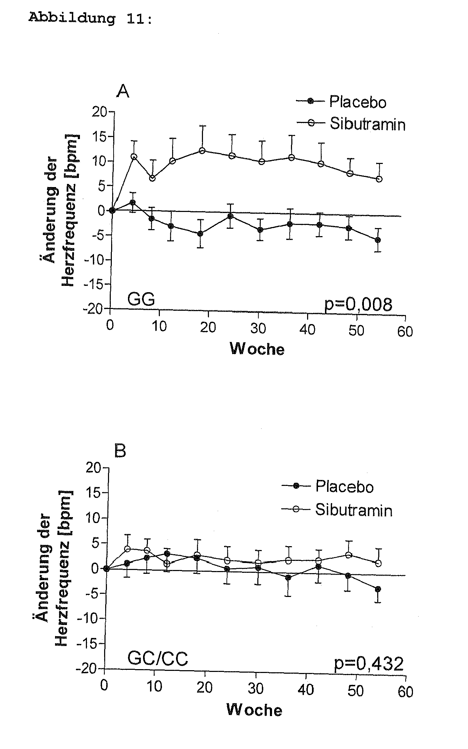

Abbildung 11 zeigt die Verwendung einer Genveränderung im humanen GNA11 Gen zur Vorhersage eines Anstiegs der Herzfrequenz unter Therapie mit Sibutramin. Dargestellt sind die Effekte in Abhängigkeit von Genotypen des G(-659)C-Polymorphismus.

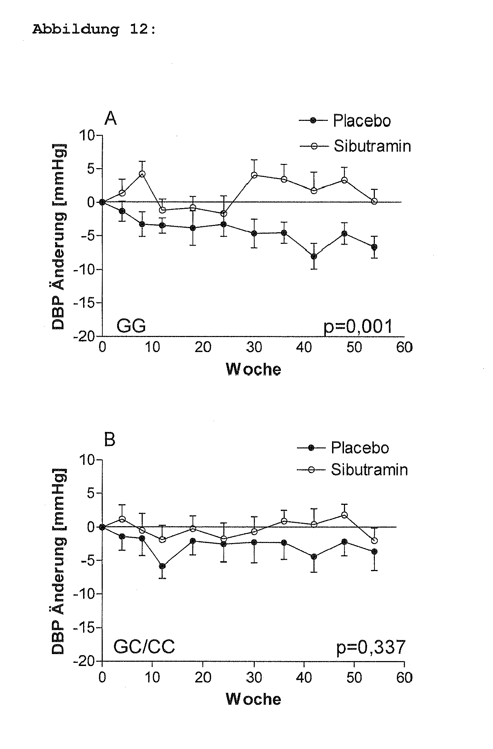

Abbildung 12 zeigt die Verwendung einer Genveränderungen im humanen GNA11 Gen zur Vorhersage eines Anstiegs des diastolischen Blutdrucks unter Therapie mit Sibutramin. Dargestellt sind die zeitabhängigen Veränderungen des diastolischen Blutdrucks (DBP) über die Zeit in Abhängigkeit von Genotypen des G(-659)C-Polymorphismus.

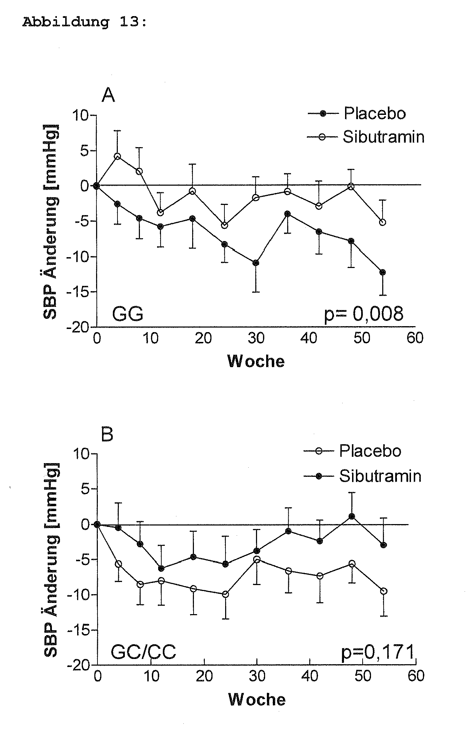

Abbildung 13 zeigt die Verwendung einer Genveränderung im humanen GNA11 Gen zur Vorhersage eines Anstiegs des systolischen Blutdrucks unter Therapie mit Sibutramin. Dargestellt sind die zeitabhängigen Veränderungen des systolischen Blutdrucks (SBP) über die Zeit in Abhängigkeit von Genotypen des G(-659)C-Polymorphismus.

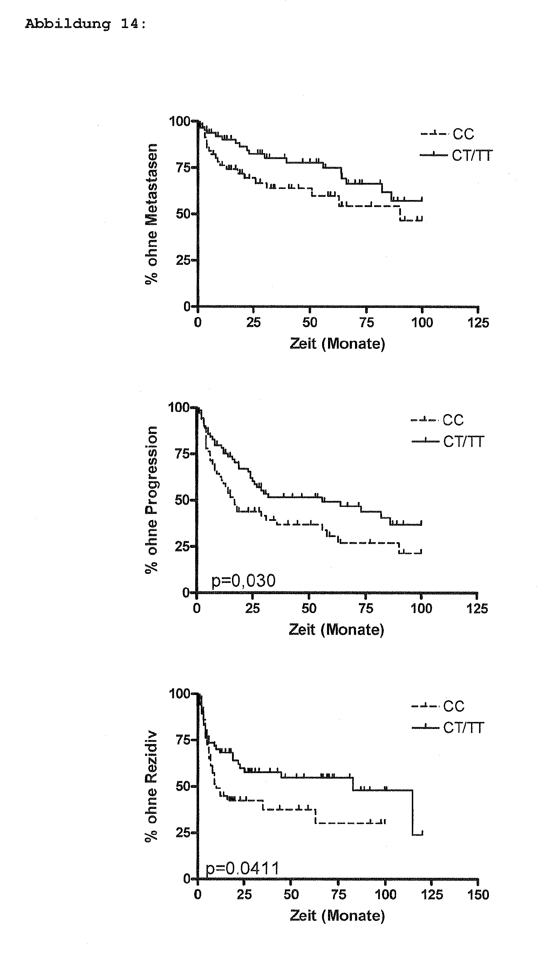

Abbildung 14 zeigt die Verwendung einer Genveränderung im humanen GNA11 Gen zur Vorhersage eines Anstiegs des systolischen Blutdrucks unter Therapie mit Sibutramin. Dargestellt sind die zeitabhängigen Veränderungen des systolischen Blutdrucks (SBP) über die Zeit in Abhängigkeit von Genotypen des G(-659)C-Polymorphismus.

Abbildung 14 zeigt einen GNA11 Intron I Polymorphismus C(10564)T und die Zeit bis zur Metastasierung (oben), bis zur Progression (Mitte) und bis zur Bildung eines Rezidivs (unten) bei Patienten mit Harnblasenkarzinom

Abbildung 15 zeigt einen GNA11 C(10564)T-Polymorphismus und Überleben bei Patienten mit Harnblasenkarzinom.

### BEVORZUGTE AUSFÜHRUNGSFORMEN DER ERFINDUNG

Das humane Gen GNA11, das für Gα11 kodiert, ist auf Chromosom 19p13.3 lokalisiert (Accession no. NW 927173 der Genbank des National Center for Biotechnology Information (NCBI)).

Zur Durchführung des erfindungsgemäßen Verfahrens geeignete Genveränderungen sind solche, die vor Exon 1 im Promotor des Gens liegend wie der Gen-Polymorphismus G(-659)C, rs11084997 (Abbildung 2), der in der Literatur noch nicht mit Assoziationen zu bestimmten Krankheiten beschrieben ist. Die Promotorsequenz wird durch den Polymorphismus in diesem Abschnitt von GCTGCCC zu GCTCCCC geändert. Zwei weitere erfindungsgemäß geeignete Gen-Polymorphismen liegen ebenfalls in der GNA11 Promotorregion bei A(-761)C (rs7245431) und bei G(-626)A (rs11084998). Die Nummerierung dieser SNPs erfolgt in der Weise, dass dem Nukleotid A des Startcodon ATG die Nummer +1 zugeordnet wird. Da es der Konvention entsprechend die Nummer 0 nicht gibt, ist dem vor dem A des Startcodon ATG liegenden Nukleotid die Nummer -1 zugeordnet.

Geeignet zur Durchführung des erfindungsgemäßen Verfahrens sind ferner Genveränderungen in Intron 1 des humanen GNA11 Gens. So der G(1606)T - Polymorphismus, so dass eine Teilsequenz von Intron 1 von AAGGGTT zu AAGTGTT geändert wird. Daneben wurde in Intron 1 ein C(10564)T - Polymorphismus detektiert (rs 3968546), so dass eine Teilsequenz von Intron 1 von GGCCGGA zu GGCTGGA geändert wird. Die Nummerierung dieser beiden SNPs erfolgt in der Weise, dass dem Nukleotid A des Startcodon ATG die Nummer +1 zugeordnet wird und über das Exon hinaus in den Intronbereich fortlaufend gezählt wird.
Die erfindungsgemäß aufgefundenen Polymorphismen im Gen GNA11 stehen untereinander in einem Kopplungsungleichgewicht und können allein oder in beliebigen Kombinationen zur Vorhersage von Krankheitsverläufen oder Wirkungen von Pharmaka verwendet werden. Der Erfindung bezieht sich somit einerseits auf den Nachweis der Genveränderungen und von Haplotypen oder Kopplungsungleichgewichten. Die Erfindung bezieht sich andererseits auf die Verwendung der ermittelten Genveränderungen zur Vorhersage von Erkrankungsrisiken und Krankheitsverläufen. Grundlage dafür ist die geänderte Aktivierbarkeit von Gα11 auf Grund der beschrieben Genveränderungen. Diese bestimmen dann gleichzeitig ein vom Genstatus abhängiges unterschiedliches Ansprechen auf Pharmaka.

Das erfindungsgemäße Verfahren unter Einsatz der zuvor bezeichneten SNPs kann mit beliebigen, dem Fachmann geläufigen Verfahren erfolgen. Solche Verfahren sind beispielsweise die direkte Sequenzierung, PCR mit nachfolgender Restriktionsanalyse, reverse Hybridisierung, Dot-blot- oder Slot-blot-Verfahren, Massenspektrometrie, Taqman^{®}- oder Light-Cycler^{®}-Technologie, Pyrosequencing^{®}, Invader^{®}-Technologie, Luminex-Verfahren. Ferner können diese Genpolymorphismen gleichzeitig nach Mulitplex-PCR und Hybridisierung an einem DNA-Chip detektiert werden.

Es wurde festgestellt, dass Änderungen der Expression von Gα₁₁ (Überexpression oder fehlende Expression) im Tierexperiment oder auf zellulärer Ebene zu einer Reihe von Krankheitszuständen oder Phänotypen führt, wie
1. Die Überexpression von Gαq/11 im Herzen führt zu Hypertrophie, Herzinsuffizienz und Apoptose.
2. Konstitutiv aktives Gαq/11 hemmt die Zellproliferation und induziert Apoptose über den Proteinkinase C-Weg.
3. Knockout von Gα11 zeigt bei Mäusen weder Verhaltensstörungen noch morphologische Veränderungen, die Tiere leben und sind fertil.
4. Knockout von Gαq/11 dagegen bringt erhebliche Defekte mit sich; die Embryonen sterben durch eine Fehlentwicklung des Herzens. Ist eine Kopie des jeweiligen Gens vorhanden, sterben die Mäuse kurz nach der Geburt aufgrund von Missbildungen des Schädels oder Herzens.
5. Gα11 ist an der Signaltransduktion von Insulin beteiligt.
6. Reduzierte Expression von Gα11 und MCT1 stehen mit der Entstehung von Brustkrebs in Zusammenhang (Asada K et al. Reduced expression of GNA11 and silencing of MCT1 in human breast cancers. Oncology 2003; 64:380-388).

Bereits diese wenigen Beispiele belegen, dass funktionsverändernde Mutationen in Gal1 oder die Unter- oder Überexpression der Proteine auch beim Menschen zu unterschiedlichen Krankheiten und/oder Funktionsstörungen führen können.

Es wurde die Verteilung der Promotor-G(-659)C, Intronl-G(1606)T und Intronl-C(10564)T-Polymorphismen bei unterschiedlichen Ethnien und Verwendung dieser Genotypen zum Auffinden weiterer relevanter Polymorphismen und Haplotypen. Dazu wurden unterschiedliche DNA-Proben von Kaukasiern, Schwarzafrikanern und Chinesen genotypisiert. Das Ergebnis ist in den folgenden Tabellen dargestellt.

| **G(-659)C** | **Weiße Kaukasier** | **Chinesen** | **Schwarze Afrikaner** |
|---|---|---|---|
| ***n* %** | 95 | 93 | 94 |
| **GG** | 39 (41 %) | 56 (60,2 %) | 59 (62,7 %) |
| **GC** | 48 (50,5%) | 35 (73,6 %) | 31 (33 %) |
| **CC** | 8 (8,5 %) | 2 (2,2 %) | 4 (4,3 %) |
| **%G** | 66,3 | 79,0 | 79,3 |

Diese Genotypverteilung ist im Chi²-Test mit einem Chi 13,8 und einem p < 0,008 signifikant verschieden. Der GG-Genotyp tritt bei Schwarzafrikanern am häufigsten auf.

| **Intron 1 G(1606)T** | **Weiße Kaukasier** | **Chinesen** | **Schwarze Afrikaner** |
|---|---|---|---|
| ***n*%** | 98 | 91 | 94 |
| **GG** | 32 (32,3%) | 54 (59,3%) | 75 (79,8%) |
| **GT** | 52 (52,5 %) | 35 (38,5%) | 18 (19,2 %) |
| **TT** | 15 (15,2 %) | 2 (2,2%) | 1 (1 %) |
| **%G** | 59,2 | 78,6 | 89,4 |

Diese Genotypverteilung ist im Chi²-Test mit einem Chi 51,0 und einem p < 0.0001 hoch signifikant verschieden. Der GG-Genotyp tritt bei Schwarzafrikanern am häufigsten auf.

| **Intron 1 C(10564)T** | **Weiße Kaukasier** | **Chinesen** | **Schwarze Afrikaner** |
|---|---|---|---|
| ***n*%** | 98 | 84 | 86 |
| **CC** | 46 (46,5 %) | 28 (33,3%) | 39 (45,3%) |
| **CT** | 43 (43,3%) | 42 (50,0%) | 36 (41,9%) |
| **TT** | 10 (10,1%) | 14 (16,7%) | 11 (12,8%) |
| **%C** | 68,2 | 58,3 | 66,3 |

Diese Genotypverteilung zeigt im Chi² -Test mit einem Chi 4,4 und einem p = 0,352 keinen signifikanten Unterschied zwischen den Ethnien. Der CC-Genotyp tritt bei Schwarzafrikanern am häufigsten auf, doch bei Chinesen sind am stärksten die heterozygoten CT-Träger vorhanden. Aus dieser Verteilung kann man folgern, dass entwicklungsgeschichtlich (bezogen auf Kaukasier) der GG(-659) und der GG(1606) den "Urzustand" darstellen. Solche Unterschiede der Genotypverteilung bei unterschiedlichen Ethnien weisen in der Regel darauf hin, dass assoziierte Phänotypen für die Evolution bedeutsam waren und den Trägern einen bestimmten Vorteil brachten. Es ist dem Fachmann bekannt, dass ethnisch unterschiedliche Genotypverteilungen ein Hinweis darauf sind, dass auch heute noch bestimmte Genotypen- und Haplotypen mit bestimmten Erkrankungen oder physiologischen und pathopyhsiologischen Reaktionsweisen oder Ansprechen auf Therapie, z.B. mit Pharmaka, assoziiert sind.

Gegenstand der Erfindung ist auch, dass diese Polymorphismen dazu benutzt werden, weitere relevante genomische Genveränderungen in GNA11 oder benachbarten Genen zu detektieren und zu validieren, die beispielsweise mit Genotypen im Gen GNA11 im Kopplungsungleichgewicht stehen. Dies können auch Gene sein, die ebenfalls auf Chromosom 19 liegen, aber in großer Entfernung vom Gen GNA11. Hierzu wird folgendermaßen vorgegangen:
1. Für bestimmte Phänotypen (zelluläre Eigenschaften, Krankheitszustände, Krankheitsverläufe, Arzneimittelansprechen usw.) wird zunächst eine Assoziation mit den Polymorphismen G(-659)C, G(1606)T oder C(10564)T hergestellt.
2. Für neu detektierte Genveränderungen in GNA11 oder benachbarten Genen wird untersucht, ob bereits bestehende Assoziationen unter Verwendung oben beschriebener Genotypen oder Haplotypen verstärkt oder abgeschwächt werden.

### Haplotypenanalyse des Promotor Polymorphismus G(-659)C mit den Intron I Polymorphismen G(1606)T und C(10564)T

Um eine Aussage darüber treffen zu können, ob die drei oben genannten Polymorphismen miteinander vererbt werden und in welchem Kopplungsungleichgewicht sie untereinander stehen, wurde eine Haplotypenanalyse mit dem Computer-Programm "Haploview" durchgeführt. Dabei wurde deutlich, dass der Promotor Polymorphismus G(-659)C zu 82% an den G(1606)T und zu 75% an den C(10564)T koppelt. Die beiden Intron I Polymorphismen G(1606)T und C(10564)T koppeln zu 77% aneinander (Abbildung 3). Das bedeutet, dass es nicht immer ausreichend ist, nur einen der drei genannten Polymorphismen zu untersuchen, um dann Rückschlüsse auf die anderen ziehen zu können. Dazu wäre eine nahezu 100%ige Kopplung nötig.

### Funktionelle Bedeutung des G(-659)C Polymorphismus

Es wurde untersucht, welche funktionellen Änderungen den Genotypen der hier beschriebenen Polymorphismen im Gen GNA11 zuzuordnen sind. Denkbar ist hier beispielsweise eine gewebespezifische Expression oder eine Überexpression des Gα11-Proteins in Abhängigkeit von Genotypen des G(-659)C Polymorphismus oder der anderen genannten Polymorphismen. Hierzu wurde zunächst mittels eines Computer-Programms untersucht, ob der Nukleotidaustausch die Bindung von Transkriptionsfaktoren beeinflussen kann.

Transkriptionsfaktoren binden an spezifische Konsensus-Sequenzen und können die Promoteraktivität steigern oder vermindern, so dass eine verstärkte oder verminderte Transkription des Gens resultiert und somit das Expressionsniveau des kodierten Proteins gesteigert oder vermindert wird. Der G(-659)C-Polymorphismus befindet sich in einer Konsensus-Sequenz der Bindungsstelle für die Transkriptionsfaktoren AP2-alpha, Myf, SPI-1 und MZF 1-4, deren Bindungsfähigkeit durch den Polymorphismus beeinträchtigt werden kann. Diese Beeinträchtigung bezieht sich hauptsächlich auf die Bindungsfähigkeit des Transkriptionsfaktors AP2-alpha auf den Genotyp G(-659). Das Auftreten dieses Genotyps führt zu einer schwächeren Bindung oder eines Wegfalls an die Konsensus-Sequenz: TCCCCAGGC. AP2-alpha kann hemmend auf die Expression von Genen wirken (Jiang et al., The repressive function of AP2 transcription factor on the hepatocyte growth factor gene promoter. Biochem Biophys Res Commun. 2000 Jun 16;272(3):882-6; Ren and Liao, Transcription factor AP-2 functions as a repressor that contributes to the liver-specific expression of serum amyloid A1 gene. J Biol Chem. 2001 May 25;276(21):17770-8.

Zur experimentellen Untersuchung dieses Effekts wird ein sogenannter EMSA (electrophoretic mobility shift assay) durchgeführt. Bei diesem Versuch werden kurze Nukleinsäureabschnitte, die den Polymorphismus beinhalten, mit Zellkernextrakten inkubiert. Transkriptionsfaktor-Proteine, die sich in diesen Extrakten befinden, binden nun mit unterschiedlicher Intensität an die Nukleinsäureabschnitte. Die Bindung an die DNA wird schließlich im Röntgenfilm sichtbar gemacht. Dabei resultiert aus einer starken Bindung eine intensive Bande. Abbildung 4 zeigt das Resultat dieses Versuches mit spezifischen Konstrukten, die entweder den G- oder den C-Genotyp enthalten. Die stärkere Intensität der C-Konstruktbande beweist eine stärkere Bindung eines Transkriptionsfaktors an diese Region.

Zum funktionellen Nachweis einer geänderten Promotoraktivität abhängig von bestimmten Genotypen wurden unterschiedliche Fragmente des Promoters mit dem G- oder mit dem C-Genotyp in den Vektor pSEAP kloniert, um nach Expression des Vektors in HEK-Zellen die Promotoraktivität mittels eines sogenannter "Reporterassays" zu quantifizieren (Abbildung 5). Hierzu werden die Konstrukte vor ein Gen kloniert, das für sezernierte alkalische Phosphatase (SEAP) kodiert. Falls das Konstrukt eine Promoteraktivität hat, wird das SEAP-Gen vermehrt transkribiert und die vermehrte Sekretion von alkalischer Phosphatase ins Zellkulturmedium ist messbar. Wie Abbildung 5 zeigt, weist das Konstrukt -849/-274 eine hohe Promotoraktivität auf. Da sich der Polymorphismus in dieser Region befindet und außerdem eine Transkriptionsfaktor Bindungsstelle durch den Nukleinsäureaustausch beeinflusst wird, wurde nun untersucht, inwiefern sich die Promotoraktivität des Konstruktes in Abhängigkeit des Genotyps verhält.

Hierfür wurde das Konstrukt -849/-274 mit jeweils dem Alle1 G(-659) und C(-659) in HEK- und Hela-Zellen transfiziert. Bei Konstrukten mit dem G-Allel ist die Promotoraktivität 4-5fach signifikant (p < 0.05) im Vergleich zum C-Allel gesteigert. Der G-Polymorphismus im Promotor des GNA11-Gens führt also dazu, dass die Promotoraktivität gesteigert ist und demnach das Gα11-Protein vermehrt exprimiert wird.

Um zu überprüfen, ob diese Regulation auch in vivo stattfindet, wurde die Expression von Gα11 auf mRNA-Ebene mittels Realtime-PCR in Harnblasentumoren untersucht. Dazu wurde mRNA aus menschlichem Operationsgewebe bei Operationen gewonnen und mittels reverser Transkriptase in cDNA umgeschrieben. Das Verfahren ist dem Fachmann geläufig. Nachfolgend wurde das Expressionsniveau mittels Realtime-PCR (Taqman-Verfahren) bestimmt und mit dem Expressionsniveau des Housekeeping-Gens β-Actin abgeglichen. Die Ergebnisse sind in Abbildung 6 dargestellt. Der GG Genotyp führt zu einer Steigerung der Gα11 Transkription von mindestens 20% gegenüber dem C-Allel.

Das erfindungsgemäße Verfahren findet Anwendung in der Vorhersage, Diagnose und Therapie folgender Krankheiten. Diese Krankheiten gehen einher mit einer verändertem Expressionsniveau von Gα11-Protein.
1. Herz-Kreislauferkrankungen. Darunter fallen insbesondere Hypertonie, Schlaganfall, koronare Herzkrankheit und Myokardinfarkt, Herzinsuffizienz, Herzrhythmusstörungen, Präeklampsie bzw. Gestose,
2. Enokrinologische und Stoffwechselerkrankungen. Darunter fallen insbesondere Adipositas, metabolisches Syndrom, Typ-2 Diabetes-mellitus, Gicht, Osteoporose, Schilddrüsenerkrankungen wie Hyper- und Hypothyreose und M. Basedow, Hyper- und Hypoparathyreodismus, Morbus Cushing, Hyper- und Hypoaldosteronismus,
3. Psychiatrische Erkrankungen wie Depression, Schizophrenie, Alkoholismus und Angststörungen, Phobien, Neurosen,
4. Neurologische Erkrankungen wie Morbus Parkinson, multiple Sklerose, Epilepsien,
5. Dermatologische Erkrankungen wie Psoriasis, Neurodermitis,
6. Tumorerkrankungen.

Beispielhaft wird nachfolgend die Verwendung von Genveränderungen im Gen GNA11 zur Vorhersage des Risikos für Herz-Kreislauferkrankungen erörtert.

Bei transgenen Tieren, die im Herzen Gα/11 überexprimieren beobachtet man eine vermehrte Neigung zur Herzhypertrophie, Herzinsuffizienz und Apoptose. Es ist daher davon auszugehen, dass eine gesteigerte Expression von Gα11 beim Menschen, wie sie beim GG-Genotyp des G(-659)C-Polymorphismus auftritt, zu einem gesteigerten kardiovaskulären Risiko führt.

Wie in Abbildung 7 dargestellt, ist der pulmonalarterielle Mitteldruck bei Patienten mit koronarer Herzerkrankung, die für den GG-Genotyp homozygot sind signifikant (p=0.006) im Vergleich zum CC-Genotyp erhöht. Auch für den pulmonalvaskulären Widerstand (Abbildung 8) konnte ein nahezu signifikanter Unterschied (p=0,062) zwischen dem GG- und dem CC-Genotyp ermittelt werden. Bei Betrachtung der GC/CC-Allelträger in Kombination wurden die Werte mit p=0,026 signifikant. Bei der Echokardiographie lassen sich deutliche genotypabhängige Unterschiede in der Auswurfsfraktion bei Patienten zeigen, die bereits einen Herzinfarkt erlitten haben (p=0,047)(Abbildung 9 A und B). Somit besteht für C-Allelträger nach einem Herzinfarkt mit einer Auswurfsfraktion von etwa 52% eine bessere Prognose, als für G-Allelträger mit einer eingeschränkten Auswurfsfraktion von knapp 43%. Insgesamt lässt sich damit den (-659)G-Allelträgern ein deutlich erhöhtes kardiovaskuläres Risiko zuordnen.

Das erfindungsgemäße Verfahren kann zu pharmakogenetischen Zwecken verwendet werden, d.h. zur Diagnostik der Wirksamkeit von Pharmaka, der Potenz und Effizienz von Pharmaka und dem Auftreten unerwünschter Wirkungen.

Die Wirksamkeit von Arzneimitteln und/oder das Auftreten unerwünschter Nebenwirkungen wird neben den spezifischen Stoffeigenschaften der chemisch definierten Produkte durch eine Reihe von Parametern definiert. Zwei wichtige Parameter, die erzielbare Plasmakonzentration und die Plasmahalbwertszeit bestimmen in hohem Maße die Wirksamkeit oder Unwirksamkeit von Pharmaka oder das Auftreten unerwünschter Wirkungen. Die Plasmahalbwertszeit wird unter anderem dadurch bestimmt mit welcher Geschwindigkeit bestimmte Pharmaka in der Leber oder anderen Körperorganen zu wirksamen oder unwirksamen Metaboliten verstoffwechselt und mit welcher Geschwindigkeit sie aus dem Körper ausgeschieden werden, wobei die Ausscheidung über die Nieren, über die Atemluft, über den Schweiß, über die Spermaflüssigkeit, über den Stuhl oder über andere Körpersekrete erfolgen kann. Daneben wird die Wirksamkeit bei oraler Gabe durch den sogenannten "first-pass-Effekt" limitiert, da nach Resorption von Pharmaka über den Darm ein bestimmter Anteil in der Leber zu unwirksamen Metaboliten verstoffwechselt wird.

Mutationen oder Polymorphismen in Genen metabolisierender Enzyme können die Aktivität derselben in der Weise verändern, dass deren Aminosäurezusammensetzung verändert wird, wodurch die Affinität zum metabolisierenden Substrat erhöht oder erniedrigt wird und damit der Metabolismus beschleunigt oder verlangsamt sein kann. In ähnlicher Weise können Mutationen oder Polymorphismen in Transportproteinen die Aminosäurezusammensetzung in der Weise verändern, dass der Transport und damit die Ausscheidung aus dem Körper beschleunigt oder verlangsamt wird. Zur Auswahl der für einen Patienten optimal geeigneten Substanz, der optimalen Dosierung, der optimalen Darreichungsform und zur Vermeidung unerwünschter, teils gesundheitsschädlicher oder tödlicher Nebenwirkungen ist die Kenntnis von genetischen Polymorphismen oder von Mutationen, die zur Änderung der Genprodukte führen, von herausragender Bedeutung.

### Die Wirkung von Hormonen im menschlichen Körper und die Bedeutung von Polymorphismen in Hormonrezeptoren

Ein Vielzahl von Hormonen und Peptidhormonen des menschlichen Körpers aber auch Rezeptorantagonisten üben ihre Wirkung an sogenannten Rezeptoren der Körperzellen aus. Dies sind Proteine unterschiedlicher Zusammensetzung. Nach Aktivierung dieser Rezeptoren müssen diese Signale ins Zellinnere geleitet werden, was über die Aktivierung von heterotrimeren G-Proteinen vermittelt wird. Solche G-Proteine sind aus unterschiedlichen α-, β-und γ- Untereinheiten zusammengesetzt. Diese Rezeptoren lassen sich, je nach Aktivierbarkeit durch definierte Hormone, in bestimmte Gruppen unterteilen. Dem Fachmann ist bekannt, dass Mutationen oder Polymorphismen in bestimmten Rezeptoren die Wirksamkeit bestimmter Agonisten oder Antagonisten an diesen Rezeptoren determinieren können. So beeinflusst ein häufiger Gly16Arg- Polymorphismus im Gen, das für den β2-Adrenozeptor kodiert, die Stärke der Ansprechbarkeit auf das β2-Sympathomimetikum Salbutamol (Martinez FD, et al. Association between genetic polymorphisms of the beta2-adrenoceptor and response to albuterol in children with and without a history of wheezing. J Clin Invest. 1997 Dec 15;100(12):3184-8). Polymorphismen im D2-Rezeptorgen bestimmen die Häufigkeit des Auftretens von Dyskinesien bei der Behandlung des Morbus Parkinson (Parkinson's disease) mit Levadopa (Oliveri RL, et al.; Dopamine D2 receptor gene polymorphism and the risk of levodopainduced dyskinesias in PD. Neurology. 1999 Oct 22;53(7):1425-30): Polymorphismen im *µ*-Opiatrezeptor-Gen bestimmen die analgetische Wirksamkeit von Opiaten (Uhl GR, et al. The mu opiate receptor as a candidate gene for pain: polymorphisms, variations in expression, nociception, and opiate responses. Proc Natl Acad Sci USA. 1999 Jul 6;96(14):7752-5).

Die genannten Genveränderungen in spezifischen Rezeptoren können nur in der Weise zur Diagnostik der Wirkungen von Pharmaka benützt werden, als diese Pharmaka spezifische Agonisten oder Antagonisten an den betrachteten Rezeptoren sind. Wünschenswert ist hingegen eine individuelle Diagnostik der generellen Ansprechbarkeit gegenüber allen Pharmaka und die individuelle Vorhersage des Risikos unerwünschter Wirkungen unter Therapie mit Pharmaka.

Die Diagnostik der Aktivierbarkeit des Gα11-Proteins erlaubt eine generelle Diagnostik der Wirksamkeit von Pharmaka, deren optimale Dosierung und das Auftreten von Nebenwirkungen.

Unter Pharmaka verstehen wir generell Stoffe, die dem menschlichen Körper von außen zugeführt werden, um bestimmte Zustände herzustelle. Solche Stoffe können Hormone, nieder- oder hochmolekulare Substanzen, Peptide- oder Proteine, Antikörper sein.

Die meisten zur Behandlung von Krankheiten, körperlichen Fehlfunktionen oder Befindlichkeitsstörungen eingesetzten Pharmaka sind Hormone, Agonisten an Hormonrezeptoren, Antagonisten an Hormonrezeptoren oder andere Substanzen, welche die Expression von Rezeptoren oder die Konzentration von Hormonen direkt oder indirekt beeinflussen. Eine Reihe von Pharmaka übt diesen Einfluss dadurch aus, dass unter Therapie mit solchen Substanzen physiologische Gegenregulationen erfolgen, welche die Konzentrationen von Hormonen erhöhen, die G-Protein-gekoppelte Rezeptoren aktivieren. Als allgemein bekanntes Beispiel sei hier die Therapie mit harntreibenden Substanzen (Diuretika), insbesondere Schleifendiuretika und Thiaziddiuretika genannt. Der bei Therapie auftretende Verlust von Kochsalz und die Blutdrucksenkung führen zur Aktivierung des Renin/Angiotensin/Aldosteronsystems. Das vermehrt gebildete Hormon Angiotensin II stimuliert eine vermehrte Resorption von Natrium in der Niere, stimuliert die Salzaufnahme, erhöht den Blutdruck durch einen direkten vasokonstriktorischen Effekt auf glatte Gefäßmuskelzellen und induziert Proliferationsvorgänge. Es ist allgemein bekannt, dass diese von Angiotensin II hervorgerufenen Mechanismen nach Kopplung des Hormons an Rezeptoren erfolgt, welche Ihre Wirkung über eine Aktivierung heterotrimerer G-Proteine vermitteln. Die Effizienz dieser Wirkungen sind dann vorhersagbar, wenn die Stärke der Aktivierbarkeit von G-Proteinen diagnostiziert werden kann. Andere Pharmaka üben Ihre Wirkung dadurch aus, dass sie die Wiederaufnahme von aus Neuronen freigesetzten Transmittern, z.B. Noradrenalin, Adrenalin, Serotonin oder Dopamin, hemmen. Hier kann als Beispiel das Pharmakon Sibutramin genannt werden, welches im Zentralnervensystem die Wiederaufnahme von Serotonin und Noradrenalin hemmt, dadurch das Hungergefühl reduziert und die Thermogenese steigert. Entsprechend kann Sibutramin zur Therapie der Adipositas eingesetzt werden. Da Noradrenalin und Serotonin G-Protein-gekoppelte Rezeptoren aktivieren, ist die Diagnostik der Aktivierbarkeit von G-Proteinen zur Vorhersage der Wirksamkeit von Sibutramin und dem Auftreten typischer, Sibutramin-assoziierter Nebenwirkungen (z.B. Anstieg von Herzfrequenz und Blutdruck) in besonderem Maße geeignet.

Erfindungsgemäß wird nun ein Verfahren bereitgestellt, das zur generellen Diagnostik der Aktivierbarkeit von G-Proteinen geeignet ist. Hierzu werden eine oder mehrere Polymorphismen im Gen GNA11 untersucht, das für die humane Gα11-Untereinheit heterotrimerer G-Proteine kodiert. Bei Überexpression kommt es vorhersagbar zu einer gesteigerten Aktivierbarkeit von heterotrimeren G-Proteinen und zur verstärkten Aktivierbarkeit aller Zellen des menschlichen Körpers. Damit erlaubt eine Bestimmung des Vorliegens von Polymorphismen in GNA11 die Diagnostik der Wirksamkeit und unerwünschten Wirkungen von Arzneimitteln, insbesondere Agonisten und Antagonisten aller Rezeptoren, deren Wirkungen über heterotrimere G-Proteine vermittelt werden. Daneben können solche Polymorphismen in GNA11 dazu verwendet werden, die Wirkungen von Pharmaka zu diagnostizieren, die entweder indirekt oder infolge von Gegenregulationsmechnanismen des Körpers die Konzentrationen von endogenen Hormonen erhöhen oder erniedrigen, deren Rezeptoren heterotrimere G-Proteine aktivieren. Somit erlaubt die Erfindung eine Diagnostik von Wirkungen und unerwünschten Wirkungen aller Pharmaka und beschränkt sich nicht auf Pharmaka die in agonistischer oder antagonistischer Weise spezifische Rezeptoren beeinflussen. Zusätzlich kann die Diagnostik des Allel- oder Haplotypstatus in GNA11 dazu eingesetzt werden, die individuell optimale und verträgliche Dosierung von Arzneimitteln zu ermitteln.

Zur Diagnostik einer gesteigerten oder reduzierten Aktivierbarkeit von G-Proteinen dient insbesondere der Nachweis des G(-659)C-Polymorphismus. Daneben können zur Diagnostik alle weiteren Genveränderungen in GNA11 verwendet werden, die in einem Kopplungsungleichgewicht zu diesem Polymorphismus stehen oder die die Expression zusätzlich fördern oder hemmen.

Das genannte Verfahren eignet sich insbesondere zur Diagnostik der Wirkung von Agonisten oder Antagonisten an Rezeptoren, deren Wirkungen bekanntermaßen von G-Proteinen vermittelt werden. Hierzu werden die folgenden Beispiele genannt, wobei die Liste der Beispiele nicht abschließend ist:
- 1.: Adrenerge Rezeptoren, inbesondere α- und β-Adrenozeptoren und deren Isoformen und Untergruppen, d.h. α1- und α2-Adrenozeptoren sowie β1-, β2-, β3 und β4-Adrenozeptoren.
- 2.: Muskarinrezeptoren und deren Isoformen, z.B. ml-,m2-, m3-, m4 und m5-Muskarinrezeptoren und deren Subtypen. Typische Antagonisten an Muskarinrezeptoren sind beispielsweise Atropin, Scopolamin, Ipratroprium, Pirenzepin und N-Butylscopolamin. Typische Agonisten sind Carbachol, Bethanechol, Pilocarpin.
- 3.: Dopaminrezeptoren, z.B. D1-, D2-, D3-, D4-, und D5-Rezeptoren und deren Isoformen und Spleißvarianten.
- 4.: Serotoninrezeptoren, z.B. 5-HT1- 5-HT2-, 5-HT3-, 5-HT4-, 5HT-5, 5HT-6 und 5-HT7-Rezeptor und deren Subtypen, Isoformen und Spleißvarianten. Typische Agonisten sind Sumatriptan und Cisaprid, Antagonsiten sind beispielsweise Ondansetron, Methysergid, Buspiron und Urapidil.
- 5.: Endothelinrezeptoren und deren Subtypen, Isoformen und Spleißvarianten.
- 6.: Bradykininrezeptoren, wie B1- und B2-Rezeptoren und deren Subtypen, Isoformen und Spleißvarianten.
- 7.: Angiotensinrezeptoren, z.B. AT II Typ1 und Typ2 Rezeptor, typische Antagonisten am AT II-Rezeptor sind Losartan und andere Sartane.
- 8.: Rezeptoren für Endorphine und Opiate, wie der *µ-*Opiatrezeptor.
- 9.: Chemokinrezeptoren CCR1-12 und CXCR1-8 für z.B. Interleukin-1/2/3/4/5/6/7/8/9/10/11/12, RANTES, MIP-1α, MIP-1β, stromal cell-derived factor, MCP1-5, TARC, Lymphotactin, Fractalkine, Eotaxin 1-2, NAP-2, LIX.
- 10.: Adenosinrezeptoren und deren Subtypen, Isoformen und Spleißvarianten.
- 11.: Rezeptoren für Thrombin (Protease-aktivierte Rezeptoren).
- 12.: Rezeptoren für Lyso-Phophatidsäure, Phosphatidsäure, Rezeptoren für Sphingosinphosphate und deren Derivate.
- 13.: Rezeptoren für Prostaglandine und Thromboxane, wie für PGE1, PGE2, PGF, PGD2, PGI2, PGF2α, Thromboxan A2.
- 14.: Rezeptoren für Neuropetide, wie NPY1-5.
- 15.: Histaminrezeptoren, wie H1-H3-Rezeptoren.
- 16.: Rezeptoren für Plättchen-aktivierender Faktor (PAF-Rezeptor).
- 17.: Rezeptoren für Leukotriene.
- 18.: Rezeptoren für Insulin, Glucagon, Insulin-like growth factor (IGF-1 und IGF-2), Epidermal Growth Factor (EGF) und platelet-derived growth factor (PDGF).
- 19.: Rezeptoren für Wachstumshormon (GH), Somatostatin (SSTR1-5), thyreotropes Hormon (TSH), Oxytocin, Prolactin, Gonadotropine.
- 20.: Rezeptoren für Zytokine, wie Interferone.
- 21.: Rezeptoren für Purine.
- 22.: Orphanrezeptoren, deren Wirkungen durch G-Proteine vermittelt werden.
- 23.: Rezeptoren für Leptin.
- 24.: CpG - Oligonucleotide.

Vorhergesagt werden können ferner die Wirkungen von Pharmaka, welche die Wiederaufnahme, den Abbau oder die Neusynthese von Neurotransmittern beeinflussen oder bei denen unter Therapie Veränderungen bei der Expression oder Ansprechbarkeit der oben genannten Rezeptoren auftreten (z.B. Sibutramin, Fluoxetin). Diagnostiziert werden können außerdem die Wirkungen aller Pharmaka, welche auf direktem oder indirektem Wege auch in Folge einer physiologischen Gegenreaktion die Konzentrationen von Agonisten, welche die oben genannten Rezeptoren aktivieren, verändern. Vorausgesagt werden kann ferner der Einfluss der Strahlentherapie bei Krebspatienten.

Insbesondere die Wirkungen und unerwünschten Wirkungen der folgenden Pharmaka aus den folgenden Indikationsbereichen können diagnostiziert werden:
- 1.: Antihypertensiva, z.B. β-Blocker (Propanolol, Bisprolol, etc.), Diuretika (Hydrochlorothiazid und weitere Thiaziddiuretika; Furosemid, Piretanid und weitere Schleifendiuretika, Chlorthalidon), α1-Adrenozeptorblocker (z.B. Doxazosin, Prazosin), Angiotensinrezeptor-Blocker (z.B. Losartan), ACE-Hemmer (Enalapril, Captopril, Ramipril etc.), Ca²⁺-Kanalblocker (z.B. Nifedipin, Verapamil, Amlodipin, Felodipin), Clonidin, Reserpin, Renin- Inhibitoren.
- 2.: Pharmaka zur Behandlung der Herzinsuffizienz, z.B. β-Blocker (z.B. Propanolol, Metoprolol), ACE-Hemmer (z.B. Captopril, Enalapril, Ramipril, etc.), Angiotensin-Rezeptorblocker (z.B. Losartan), Digitalisglykoside, Katecholamine, Diuretika.
- 3.: Pharmaka zur Behandlung des niedrigen Blutdrucks oder einer Herzinsuffizienz, z.B. α- und β-Sympathomimetika (Effortil, Adrenalin, Noradrenalin, Dobutamin, β-Adrenozeptorblocker, ACE-Hemmer, Angiotensin II-Rezeptorblocker.)
- 4.: Pharmaka zur Behandlung der Migräne, z.B. Sumatriptan, Rizatriptan, Zolmitriptan und weitere Agonisten an Serotoninrezeptoren, β-Blocker (Propanolol, Timolol), Ergotamin und Dihydroergotamin.
- 5.: Analgetika vom Morphintyp (Morphin, Codein, etc.).
- 6.: Pharmaka zur Behandlung der koronaren Herzkrankheit wie Adenosin, β-Blocker (z.B. Propanolol, Acebutolol), Nitrate und Ca²⁺-Kanalblocker.
- 7.: Pharmaka zur Behandlung psychiatrischer Erkrankungen (Schizophrenie, manisch-depressive Erkrankungen, Psychosen, Depressionen) und von Suchtkrankheiten wie Alkoholismus, (z.B. Fluoxetin, Paoxetin, Imipramin, Desipramin, Doxepin, Mianserin, Trazodon, Lofepramin), Angstsyndrome (Diazepam, etc.), welche z.B. das dopaminerge, serotonerge oder adrenerge System beeinflussen. Aber auch Pharmaka, die ihre Wirkung über Rezeptoren für GABA-, Glycin- oder Glutamat bzw. deren Derivate vermitteln.
- 8.: Pharmaka zur Behandlung des Morbus Alzheimer (z.B. Tacrin) und zur Behandlung des Morbus Parkinson (z.B. Bromocriptin, L-DOPA, Carbidopa, Biperiden, Seleginil, etc.) welche Transmitterkonzentrationen an z.B. muskarinergen oder dopaminergen Substanzen, beeinflussen.
- 9.: Pharmaka zur Behandlung von Asthma bronchiale, welche z.B. entweder direkt bronchodilatierend oder antiinflammatorisch wirken, z.B. Salbutamol, Terbutalin, Albuterol, Theophyllin, Montelukast, Zafirlukast, Cromoglicinsäure, Ipratropiumbromid. Zu solchen Pharmaka gehören auch Antikörper die gegen bestimmte Proteine und Rezeptoren gerichtet sind.
- 10.: Pharmaka zur Behandlung von Motilitätsstörungen des Magens oder Darms und Pharmaka zur Behandlung des Reizdarmsyndroms (irritable bowel syndrome) z.B. N-Butylscopolamin, Pirenzepin, Metoclopramid).
- 11.: Pharmaka zur Behandlung der Adipositas, welche entweder direkt lipolytisch wirksame Rezeptoren aktivieren, z.B. β3-adrenerge Agonisten, oder zentral wirksam sind, z.B. Sibutramin, oder ähnliche Substanzen, die das Sättigungsgefühl verändern oder welche die Thermogenese beeinflussen. Dazu gehören auch Pharmaka, welche die Magenentleerung beeinflussen.
- 12.: Pharmaka zur Behandlung chronischer Entzündungsvorgänge oder von Störungen des Immunsystems, z.B. Zytokine (Interferone) bei der Therapie von Virushepatitiden oder Interleukin-2 bei HIV-Infektion. Zu solchen Erkrankungen zählen auch Morbus Crohn, Colitis ulcerosa, Asthma, Psoriasis, Neurodermitits, Heuschnupfen. Dazu gehören auch Antikörper gegen Zytokine oder gegen Zytokinrezepotren, z.B. gegen TNFá.
- 13.: Pharmaka zur Behandlung der Gestose und der Präeklampsie/Eklampsie und des HELLP-Syndroms.
- 14.: Pharmaka zur Behandlung von Fertilitätsstörungen oder zur Beseitigung von Zyklusstörungen bei der Frau oder zur Empfängnisverhütung.
- 15.: Pharmaka zur Behandlung von Herzrhythmusstörungen.
- 16.: Antidiabetika (Acarbose, Insulin, Troglitazone, Metformin, etc.).
- 17.: Hypnotika, Antiemetika und Antiepileptika.
- 18.: Pharmaka zur Behandlung von Störungen des Sexuallebens, z.B. der erektilen Dysfunktion, der weiblichen sexuellen Dysfunktion, Libidomangel, Orgasmusstörungen (Phosphodiesteraseinhibitoren wie Sildenafil, Prostaglandin EI, Agonisten an Dopamin-Rezeptoren, z.B. Apomorphin, Yohimbin, Phentolamin).
- 19.: Pharmaka zur Therapie von Krebserkrankungen und Chemotherapeutika, z.B. 5-Fluoruracil, Antikörper gegen Proteine und Rezeptoren (z.B. gegen HER-2), Substanzen, die Tyrosinkinasen blockieren, etc.
- 20.: Pharmaka zur Behandlung von allergischen und Tumorerkrankungen, bei denen die Wirkung über die Verabreichung von CpG-Nukleotiden erzielt wird.
- 21.: Pharmaka zur Behandlung des Adipositas, des metabolischen Syndroms oder des Diabetes, z.B. Sibutramin, Orlistat, Leptin, Topiramat, Glinide, Glitazone, Biguanide etc.
- 22.: Pharmaka zur Behandlung der HIV-Infektion, auch Antikörper und Rezeptorblocker. Vorhersage des Entstehens einer Lipodystrophie unter Therapie mit Proteinaseinhibitoren.

Selbstverständlich ist es nicht möglich, im Rahmen der hier beschriebenen Erfindung den Nachweis zu führen, dass alle Pharmakawirkungen durch den GNA11-Genstatus determiniert werden. Naturgemäß kann man auch nicht die genotypabhängigen Wirkungen von Pharmaka untersuchen, die erst in Zukunft entwickelt und eingesetzt werden. Dagegen sollen hier Beispiele für Pharmaka mit unterschiedlichen Wirkmechanismen exemplarisch gezeigt werden, so dass diese Befunde generalisiert werden können.

Beim Pharmakon (Sibutramin) wird der Effekt in der Weise ausgelöst, dass die Wiederaufnahme von Serotonin und Noradrenalin im zentralen Nervensystem blockiert wird. Dadurch wird die Konzentration dieser Transmitter im synaptischen Spalt und extrazellulär erhöht. Diese Transmitter stimulieren nachfolgend G-Protein-gekoppelte Rezeptoren. Die Substanz Sibutramin selbst stimuliert nicht solche Rezeptoren.

Ein wesentlicher Gegenstand der Erfindung ist die Bereitstellung diagnostisch relevanter Genveränderungen im Gen GNA11 als Prognosefaktor zur Vorhersage des Verlaufs für alle Tumorerkrankungen des Menschen.

Generell können alle Zellen des menschlichen Körpers maligne entarten und zu einer Krebserkrankung führen. Die hier und im weiteren gemachten Ausführungen beschreiben generelle Mechanismen der Tumorprogression, der Metastasierung und des therapeutischen Ansprechens. Insofern gelten die hier beschriebenen Mechanismen und Ansprüche für alle Tumore des Menschen, beispielsweise für die folgende Tumoren:
Tumoren des Urogenitaltrakts: Hier sind zu nennen das Harnblasenkarzinom, das Nierenzellkarzinom, das Prostatakarzinom und das Seminom.
Tumoren der weiblichen Geschlechtsorgane: Das Mammakarzinom, das Korpuskarzinom, das Ovarialkarzinom, das Zervixkarzinom.
Tumoren des Gastrointestinaltraktes: Das Mundhöhlenkarzinom,das Ösophaguskarzinom, das Magenkarzinom, das Leberkarzinom, das Gallengangskarzinom, das Pankreaskarzinom, das Kolonkarzinom, das Rektumkarzinom.
Tumore des Respirationstraktes: das Kehlkopfkarzinom, das Bronchialkarzinom.
Tumore der Haut: Malignes Melanom, das Basaliom, das T-Zell-Lymphom.
Tumorerkrankungen des blutbildenden Systems: Hodgkin- und non-Hodgkin- Lymphome, akute und chronische Leukämien etc. Tumorerkrankungen des Gehirns bzw. des Nervengewebes: Glioblastom, Neuroblastom, Medulloblastom, meningeales Sarkom, Astrozytom.

Weichteiltumore, beispielsweise Sarkome und Kopf-Hals-Tumore.

Die folgenden Beispiele dienen der weiteren Erläuterung der Erfindung.

### Beispiel 1 - Wirksamkeit von Sibutramin

Hier wurden adipöse Patienten im Rahmen einer Plazebo-kontrollierten, doppelt-blind durchgeführten Studie über ein Jahr mit 15 mg Sibutramin/Tag behandelt. Sibutramin ist ein zentral wirksamer Reuptake-Inhibitor von Noradrenalin und Serotonin, die beide ihre Wirkung über G-Protein-gekoppelte Rezeptoren entfalten. Sibutramin verstärkt das Sättigungsgefühl und erleichtert im Rahmen strukturierter Gewichtsreduktionsmaßnahmen (Steigerung der körperlichen Aktivität, Reduktion der Kalorienaufnahme) das Abnehmen. Klinisches Endziel der Studie war die Gewichtsabnahme nach einem Jahr. Der genotypabhängige Vergleich Placebo versus Sibutramin ist in Abbildung 10 dargestellt. In der Sibutramingruppe konnten Patienten mit GNA11 -659 CC Genotyp deutlich besser abnehmen (17,8%) als diejenigen mit GG bzw. GC-Genotyp (6,2%/8,7%). Die Gabe von Sibutramin führt nur bei Trägern des CC-Genotyps zu einer deutlichen (p = 0,013) Steigerung der Gewichtsabnahme, während dieser günstige Arzneimitteleffekt bei Patienten mit GG-Genotyp nicht beobachtet wurde. Damit ist eine Therapie mit Sibutramin vorwiegend bei Patienten mit CC-Genotyp angezeigt, während Patienten mit GG-Genotyp genau so gut unter Placebo (6,3%) wie unter Sibutramin (6,2%) abnehmen können.

In der Placebogruppe konnten Patienten mit GNA11 (-659)GG bzw. GC-Genotyp deutlich besser abnehmen (6,3% und 5,2%) als diejenigen mit CC-Genotyp (2,4 %). Die Gabe von Sibutramin führt nur bei Trägern des CC-Genotyps zu einer deutlichen (p = 0,013) Steigerung der Gewichtsabnahme, während dieser günstige Arzneimitteleffekt bei Patienten mit GG-Genotyp nicht beobachtet wurde. Damit ist eine Therapie mit Sibutramin vorwiegend bei Patienten mit CC-Genotyp angezeigt, während Patienten mit GG bzw. GC-Genotyp auch unter Placebo gut abnehmen können. Damit wird gleichzeitig gezeigt, dass auch die Vorhersage des Erfolgs nicht-pharmakologischer Maßnahmen zur Gewichtsreduktion unter Verwendung von Genveränderungen im Gen GNA11 möglich sind. Dazu gehören strukturierte Gewichtsabnahmeprogramme (z.B. Optifast, Weight Watchers, andere Schulungsprogramme), die Einnahme von sättigenden Quellstoffen (CM3, BMI23) oder von kalorienreduzierten Nahrungsmitteln.

### Beispiel 2 - Vorhersage von kardiovaskulären Nebenwirkungen unter Therapie mit Sibutramin

Aufgrund des Wirkmechanismus (zentrale Hemmung der Wiederaufnahme von Noradrenalin und Serotonin) kommt es unter der Einnahme von Sibutramin zu typischen Nebenwirkungen wie Mundtrockenheit, Schlafstörungen und Obstipation. Gefährlicher sind jedoch die kardiovaskulären Nebenwirkungen, z.B. Anstieg der Herzfrequenz und des Blutdrucks, die zu Tachykardien und zum Myokardinfarkt führen können. Bislang ist keine Vorhersage dazu möglich, bei welchen Personen dies auftritt.

Es wurde hier der Zusammenhang von Genveränderungen im Gen GNA11 mit der Änderung von Herzfrequenz unter Einnahme von Sibutramin versus Placebo untersucht (Abbildung 11). Man erkennt bei G(-659)C und GG-Genotypen (Abbildung 11A) eine durch Sibutramin (versus Placebo) signifikant gesteigerte Herzfrequenz (p=0,008) um > 10 (GG) bis zu 17 Schläge pro Minute. Dieser Effekt tritt bei CC- bzw. GC-Genotypen nicht auf (Abbildung 11B). Ferner beobachtet man unter Therapie mit Sibutramin einen signifikanten Anstieg des diastolischen Blutdrucks (p=0,001) bei (-659) GG Genotypen (Abbildung 12A), der bei CC-und GC-Genotypen (Abbildung 12B) nicht auftritt.

Unter Therapie mit Sibutramin beobachtet man ein fehlendes Absinken des systolischen Blutdruckes bei (-659) GG Genotypen (Abbildung 13A), der Unterschied der Blutdruckveränderung ist mit p=0,008 signifikant höher als bei GC-/CC-Genotypen (Abbildung 13B).

Diese Beobachtungen sind therapeutisch deshalb so bedeutsam, da gezeigt wurde, dass gerade Patienten mit GG Genotypen durch kalorienreduzierte Ernährung sowie Lebensstiländerung allein ausreichend Gewicht verlieren, diese also nicht von einer Therapie mit Sibutramin profitieren.

Die Verwendung von Genveränderungen ist daher dazu geeignet, Patienten zu identifizieren, bei denen unter Therapie mit Medikamenten kardiovaskuläre Nebenwirkungen auftreten. Diese Effekte können direkt verursacht werden, indem solche Medikamente Rezeptoren stimulieren, die Gα11-vermittelt eine Vasokonstriktion oder eine Herzfrequenzsteigerung hervorrufen. Dazu gehören beispielsweise Sibutramin, Triptane und Noradrenalin-/Serotonin-Wiederaufnahmehemmer. Ferner gehören dazu Medikamente, die den Abbau von Katecholaminen hemmen (MAO-Hemmer) und trizyklsiche Antidepressiva. Ferner gehören dazu Medikamente, die einen Blutdruckabfall bewirken und reflektorisch eine Aktivierung des Sympathikus induzieren (z.B. Sildenafil und andere Inhibitoren von Phosphodiesterasen, Nitrate).

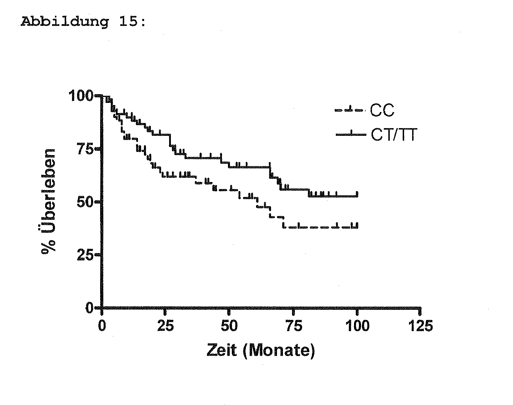

Ein weiterer Beleg für die generelle Anwendung von Genveränderungen im Gen GNA11 zur Vorhersage von Pharmakawirkungen ergibt sich auch aus den für den C(10564)T Polymorphismus beobachteten Genotyp abhängigen Krankheitsverläufen beim Harnblasenkarzinom (Abbildung 14 und 15). Diese Patienten wurden alle mit unterschiedlichen Pharmaka behandelt. Die durch Verwendung von Genveränderungen im Gen GNA11 sichtbar gemachten unterschiedlichen Krankheitsverläufe belegen ein unterschiedliches Ansprechen auf diese Therapieformen.

### Grundlegende Eigenschaften von malignen Tumoren

Bei malignen Tumoren, auch als Krebs bezeichnet, kommt es zu charakteristischen Veränderungen grundlegender Funktionen, die das Wachstum solcher Zellen in ungünstiger Weise befördern. Krebszellen sind gekennzeichnet durch einen Verlust der Kontaktinhibition und ein unkontrolliertes Zellwachstum. Ausgelöst werden solche Veränderungen durch eine Vielzahl von Noxen, sog. Kanzerogene, welche das Erbgut schädigen. Zu solchen Noxen gehören viele Chemikalien, Tabakrauch, aber auch UV-Licht. Daneben spielen genetische Faktoren bei der Krebsentstehung eine herausragende Rolle. Kennzeichnend für Krebszellen ist neben ihrem ungehemmten Wachstum auch die Neigung, Tochtergeschwülste (Metastasen) in anderen Organen abzusiedeln. Die Ausbreitung der Metastasen erfolgt regelmäßig über die Blutbahn oder über Lymphgefäße. Krebserkrankungen sind bei einem Großteil der Fälle nicht heilbar und führen zum Tode. Therapeutisch wird versucht, den Ausgangstumor und Metastasen operativ zu entfernen. Daneben können Tumore bestrahlt werden. Mittels sogenannter Zytostatika, Antikörper gegen bestimmte Proteine oder Oberflächenmarker oder immunmodulierender Substanzen (Zytokine, Interferone) wird versucht, die sich schnell teilenden Krebszellen abzutöten oder in den programmierten Zelltod (Apoptose)zu überführen. Die derzeit verfügbaren therapeutischen Maßnahmen führen in den meisten Fällen nur zu einem verlängerten Überleben, nicht zur definitiven Heilung.

### Verwendung von Genveränderungen im Gen GNA11 zur Vorhersage des Verlaufs von Tumorerkrankungen

Naturgemäß können hierbei nicht alle Tumorerkrankungen beschrieben werden. Das Prinzip wird daher an einem ausgewählten Beispiel erläutert, welche die generelle Verwendbarkeit demonstrieren:

### Beispiel 3 - Harnblasenkarzinom

Blasenkrebs ist ein bösartiger Tumor der Schleimhaut der Harnblase. Blasenkrebs tritt am häufigsten zwischen dem 60. und 70. Lebensjahr auf. Männer sind davon dreimal so häufig betroffen wie Frauen. Bei Männern ist Blasenkrebs nach Lungen- und Prostatakrebs die dritthäufigste Krebsform. Blasenkrebs kann durch äußere Einflüsse hervorgerufen werden. Zu den Risikofaktoren gehören, Rauchen, ständige Belastung des Organismus durch Chemikalien beispielsweise Farbstoffe, Schmerzmittelmissbrauch. Bei vielen Patienten ergeben die Untersuchungen, dass es sich um einen oberflächlichen Tumor handelt. Dieser kann operativ mit Hilfe des Zystoskops entfernt werden. Mehr als 70 % der Patienten, die wegen eines oberflächlichen Blasenkarzinoms behandelt wurden, zeigen im Verlauf ein Tumorrezidiv. Dabei kommen in mehr als der Hälfte Rezidivtumoren mit nichtmuskelinvasiver Erkrankung vor. Diese können durch transurethrale Resektion kurativ behandelt bzw. kontrolliert werden. Es ist deshalb wichtig, diese Läsionen frühzeitig zu erkennen und den Patienten regelmäßig und engmaschig nachzusorgen. Dabei steht an 1. Stelle die Zystoskopie mit Urinzytologie. In regelmäßigen Intervallen dienen Ausscheidungsurogramme zur Kontrolle möglicher Tumormanifestationen in Nierenbecken und Harnleitern. Bislang gibt es kaum valide Marker, die für den weiteren Verlauf der Erkrankung prädiktiv sind. Derzeit werden daher die klassischen Faktoren wie Eindringtiefe, Differenzierungsgrad, Metastasierung, Lymphknotenbefall etc. für die Prognose herangezogen. Genetische Marker für Tumorprogression, Rezidivneigung, Überlebenswahrscheinlichkeit und Therapieansprechen würden die Betreuung von Patienten mit Harnblasenkarzinom wesentlich verbessern. Ein weiterer Bestandteil der Erfindung besteht darin, dass die Verwendung von Genveränderungen in GNA11 dazu geeignet ist den weiteren Verlauf der Erkrankung vorherzusagen. Abbildung 14 (oben) zeigt den Zeitpunkt bis zum Auftreten von Metastasen in Abhängigkeit vom GNA11 C(10564)T-Polymorphismus. Hierbei ist das Metastasierungsrisiko bei Patienten mit C-Allel um ca. 1/3 erhöht. Eine ähnliche Beziehung wird gefunden, wenn die Zeit bis zur Tumorprogression untersucht wird (Abbildung 14 (Mitte)). Hierunter verstehen wir, das Auftreten von Metastasen oder das Wiederauftreten des Tumors mit höherem Staging oder Grading. Der Kurvenverlauf ist für die Genotypen signifikant verschieden (p = 0.030, Logrank-Test, wobei den CT/TT-Genotypträgern der günstigere Verlauf zugeordnet wird. Schließlich wird der Zusammenhang zwischen GNA11 C(10564)T TT-Polymorphismus und der Bildung eines Rezidivs dargestellt (Abbildung 14 unten). Auch hier wird deutlich, dass Patienten mit dem C-Allel früher ein Rezidiv entwickeln als Patienten mit dem T-Allel. In Abbildung 15 ist das Überleben über die Zeit abgebildet. Hier erkennt man wiederum, dass Patienten mit dem GNA11 CC-Genotyp früher versterben als Patienten mit dem T-Allel.

## Patentansprüche

1. *In* vitro-Verfahren zur Vorhersage von Krankheitsrisiken, Krankheitsverläufen, Arzneimittelrisiken und zum Auffinden von Drug-Targets, **dadurch gekennzeichnet, dass** man nach einer oder mehreren Genveränderung/en in der Promotorregion des Gens GNA11 und/oder im Intron 1 des Gens GNA11 auf dem Humanchromosom 19p13.3 sucht.

2. Verfahren nach Anspruch 1, **dadurch gekennzeichnet, dass** manin einer Patientenprobe nach einem Polymorphismus G(-659)C sucht.

3. Verfahren nach Anspruch 1, **dadurch gekennzeichnet, dass** man in einer Patientenprobe nach einem Polymorphismus G(1606)T sucht.

4. Verfahren nach Anspruch 1, **dadurch gekennzeichnet, dass** man in einer Patientenprobe nach einem Polymorphismus C(10564)T sucht.

5. Verfahren nach einem der Ansprüche 1 bis 4, **dadurch gekennzeichnet, dass** man in einer Patientenprobe nach zwei oder drei der Polymorphismen Promotor-G(-659)C, Intron-1G(1606)T und Intron-1C(10564)T sucht.

6. Verfahren nach Anspruch 1, **dadurch gekennzeichnet, dass** man in einer Patientenprobe nach dem Polymorphismus A(-761)C sucht.

7. Verfahren nach Anspruch 1, 2 oder 6, **dadurch gekennzeichnet, dass** man in einer Patientenprobe nach dem Polymorphismus G(-626)A sucht.
